# EUROPEAN PATENT SPECIFICATION

(11) **EP 3 734 903 B1**
(45) Date of publication and mention of the grant of the patent: **03.07.2024**
(21) Application number: 20168200.2
(22) Date of filing: 25.02.2015
(51) Int. Cl.: H04L 9/32, A61B 17/00, G01S 5/02, G01S 13/76, G01S 13/87, G06F 9/44, G08B 13/196, G08B 25/00, G08B 29/18, H04L 12/46, H04L 9/00, H04L 12/64, H04N 5/76, H04W 4/00, H04W 8/26, H04W 16/26, H04W 84/18, H04W 92/02, H04W 88/04, G07C 9/29

(54) **CORRELATION OF SENSORY INPUTS TO IDENTIFY UNAUTHORIZED PERSONS**
KORRELATION VON SENSORISCHEN EINGÄNGEN ZUR IDENTIFIZIERUNG VON UNBEFUGTEN PERSONEN
CORRÉLATION D'ENTRÉES SENSORIELLES POUR IDENTIFIER DES INDIVIDUS NON AUTORISÉS

(30) Priority: 28.02.2014 US 201461946054 P; 02.04.2014 US 201461973962 P; 20.08.2014 US 201414463765
(43) Date of publication of application: 04.11.2020
(62) Divisional of application: 15755018.7
(73) Proprietor: Tyco Fire & Security GmbH, 8212 Neuhausen am Rheinfall (CH)
(72) Inventor: LOCKE, Robert B., Sonoma, California 95476 (US)
(74) Representative: Meissner Bolte Partnerschaft mbB

(56) References cited:
- JP-A- 2005 292 942
- US-A1- 2006 059 557
- US-A1- 2007 252 001
- US-A1- 2011 001 812

## Description

This application claims priority under 35 U.S.C. §119(e) to provisional U.S. Patent Application 61/973,962, filed on April 2, 2014, entitled: "Wireless Sensor Network", and provisional U.S. Patent Application 61/946,054, filed on February 28, 2014, entitled: "Wireless Sensor Network", and utility U.S. Patent Application 14/463,765, filed on August 20, 2014, entitled: "Correlation of Sensory Inputs to Identify Unauthorized Persons".

### BACKGROUND

This description relates to operation of sensor networks such as those used for security, intrusion and alarm systems installed on commercial or residential premises.

It is common for businesses and homeowners to have a security system for detecting alarm conditions at their premises and signaling the conditions to a monitoring station or to authorized users of the security system. Security systems often include an intrusion detection panel that is electrically or wirelessly connected to a variety of sensors. Those sensors types typically include motion detectors, cameras, and proximity sensors (used to determine whether a door or window has been opened). Typically, such systems receive a very simple signal (electrically open or closed) from one or more of these sensors to indicate that a particular condition being monitored has changed or become unsecure.

Typical intrusion systems can be set up to monitor entry doors in a building.

When the door is secured, the proximity sensor senses a magnetic contact and produces an electrically closed circuit. When the door is opened, the proximity sensor opens the circuit, and sends a signal to the panel indicating that an alarm condition has occurred (e.g., an opened entry door). Government entities, companies, academic institutions, etc. issue credentials to employees, contractors, students, etc. to control access to buildings and facilities, indoors and outdoors. Individuals who bypass security systems to gain access, either intentionally or unintentionally, are difficult to identify and locate.

US 2007/252001 A1 (KAIL KEVIN J [US] ET AL) 1 November 2007 (2007-11-01

US 2006/059557 A1 (MARKHAM THOMAS R [US] ET AL) 16 March 2006 (2006-03-16

JP 2005 292942 A (SECOM CO LTD) 20 October 2005 (2005-10-20

US 2011/001812 A1 (KANG PENGJU [US] ET AL) 6 January 2011 (2011-01-06)

### SUMMARY

Prior solutions regarding credentials have focused on technologies such as video surveillance to address access problems. Once a person has gained access, however, it is difficult to impossible to distinguish between those with valid credentials and those without valid credentials.

The invention is defined by the independent claim 1. Dependent claims define preferred embodiments.

One or more aspects may provide one or more of the following advantages.

By correlating sensory input from credentials or badges that use technologies such as RFID, Bluetooth low energy (BLE), MAC addresses from cell phones, NFC, etc. with video; individuals without valid credentials can be identified and tracked. Video recognition is used to identify the number of people in a certain area. A remote reading badge is used to identify the appropriately badged employees by tracking the personnel movement and correlating that with the movement of the valid ID. Once the non-badged individual is segregated from the valid personnel, biometrics such as face, iris or just video recognition are used to track the individual(s) and allow authorities to intervene.

The details of one or more embodiments of the invention are set forth in the accompanying drawings and the description below. Other features, objects, and advantages of the invention is apparent from the claims.

### DESCRIPTION OF DRAWINGS

- FIG. 1: is a schematic diagram of an exemplary networked security system.
- FIG. 2: is a flow chart of a correlation algorithm.
- FIG. 3: is flow chart of an example tracking process.
- FIG. 4: is a block diagram of components of an example networked security system.

### DETAILED DESCRIPTION

Described herein are examples of network features that may be used in various contexts including, but not limited to, security/intrusion and alarm systems. Example security systems may include an intrusion detection panel that is electrically or wirelessly connected to a variety of sensors. Those sensors types may include motion detectors, cameras, and proximity sensors (used, e.g., to determine whether a door or window has been opened). Typically, such systems receive a relatively simple signal (electrically open or closed) from one or more of these sensors to indicate that a particular condition being monitored has changed or become unsecure.

For example, typical intrusion systems can be set-up to monitor entry doors in a building. When a door is secured, a proximity sensor senses a magnetic contact and produces an electrically closed circuit. When the door is opened, the proximity sensor opens the circuit, and sends a signal to the panel indicating that an alarm condition has occurred (e.g., an opened entry door).

Data collection systems are becoming more common in some applications, such as home safety monitoring. Data collection systems employ wireless sensor networks and wireless devices, and may include remote server-based monitoring and report generation. As described in more detail below, wireless sensor networks generally use a combination of wired and wireless links between computing devices, with wireless links usually used for the lowest level connections (e.g., end-node device to hub/gateway). In an example network, the edge (wirelessly-connected) tier of the network is comprised of resource-constrained devices with specific functions. These devices may have a small-to-moderate amount of processing power and memory, and may be battery powered, thus requiring that they conserve energy by spending much of their time in sleep mode. A typical model is one where the edge devices generally form a single wireless network in which each end-node communicates directly with its parent node in a hub-and-spoke-style architecture. The parent node may be, e.g., an access point on a gateway or a sub-coordinator which is, in turn, connected to the access point or another sub-coordinator.

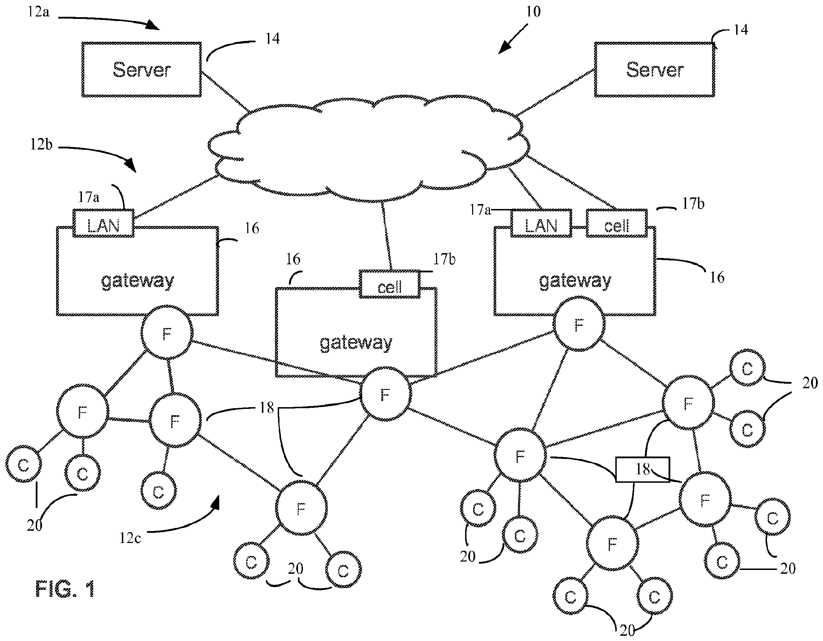

Referring now to FIG. 1, an exemplary (global) distributed network 10 topology for a Wireless Sensor Network (WSN) is shown. In FIG. 1 the distributed network 10 is logically divided into a set of tiers or hierarchical levels 12a-12c.

In an upper tier or hierarchical level 12a of the network are disposed servers and/or virtual servers 14 running a "cloud computing" paradigm that are networked together using well-established networking technology such as Internet protocols or which can be private networks that use none or part of the Internet. Applications that run on those servers 14 communicate using various protocols such as for Web Internet networks XML/SOAP, RESTful web service, and other application layer technologies such as HTTP and ATOM. The distributed network 10 has direct links between devices (nodes) as shown and discussed below.

The distributed network 10 includes a second logically divided tier or hierarchical level 12b, referred to here as a middle tier that involves gateways 16 located at central, convenient places inside individual buildings and structures. These gateways 16 communicate with servers 14 in the upper tier whether the servers are stand-alone dedicated servers and/or cloud based servers running cloud applications using web programming techniques. The middle tier gateways 16 are also shown with both local area network 17a (e.g., Ethernet or 802.11) and cellular network interfaces 17b.

The distributed network topology also includes a lower tier (edge layer) 12c set of devices that involve fully-functional sensor nodes 18 (e.g., sensor nodes that include wireless devices, e.g., transceivers or at least transmitters, which in FIG. 1 are marked in with an "F") as well as constrained wireless sensor nodes or sensor end-nodes 20 (marked in the FIG. 1 with "C"). In some embodiments wired sensors (not shown) can be included in aspects of the distributed network 10.

Constrained computing devices 20 as used herein are devices with substantially less persistent and volatile memory other computing devices, sensors in a detection system. Currently examples of constrained devices would be those with less than about a megabyte of flash/persistent memory, and less than 10-20 kbytes of RAM/volatile memory). These constrained devices 20 are configured in this manner; generally due to cost/physical configuration considerations.

In a typical network, the edge (wirelessly-connected) tier of the network is comprised of highly resource-constrained devices with specific functions. These devices have a small-to-moderate amount of processing power and memory, and often are battery powered, thus requiring that they conserve energy by spending much of their time in sleep mode. A typical model is one where the edge devices generally form a single wireless network in which each end-node communicates directly with its parent node in a hub-and-spoke-style architecture. The parent node may be, e.g., an access point on a gateway or a sub-coordinator which is, in turn, connected to the access point or another sub-coordinator.

Each gateway is equipped with an access point (fully functional node or "F" node) that is physically attached to that access point and that provides a wireless connection point to other nodes in the wireless network. The links (illustrated by lines not numbered) shown in FIG. 1 represent direct (single-hop network layer) connections between devices. A formal networking layer (that functions in each of the three tiers shown in FIG. 1) uses a series of these direct links together with routing devices to send messages (fragmented or non-fragmented) from one device to another over the network.

The WSN 10 implements a state machine approach to an application layer that runs on the lower tier devices 18 and 20. Discussed below is an example of a particular implementation of such an approach. States in the state machine are comprised of sets of functions that execute in coordination, and these functions can be individually deleted or substituted or added to in order to alter the states in the state machine of a particular lower tier device.

The WSN state function based application layer uses an edge device operating system (not shown, but such as disclosed in the above mentioned provisional application) that allows for loading and execution of individual functions (after the booting of the device) without rebooting the device (so-called "dynamic programming"). In other implementations, edge devices could use other operating systems provided such systems allow for loading and execution of individual functions (after the booting of the device) preferable without rebooting of the edge devices.

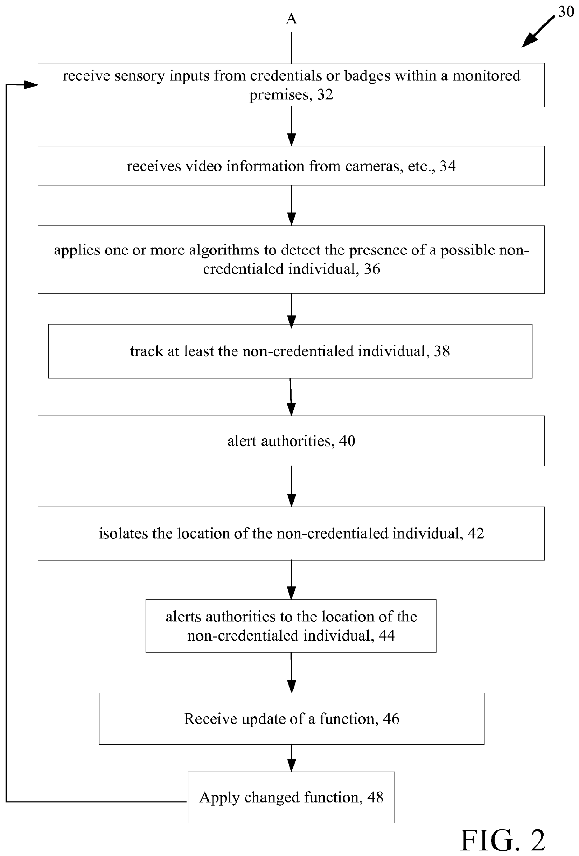

Referring now to FIG. 2 a process 30 that executes on one or more computers disposed within the distributed network 10 is shown. In alternative arrangements, the process 30 can be used in any other arrangement besides the distributed network 10 described above provided that video data and sensed credential data are supplied to the computer(s). The process 30 correlates sensory input from credentials or badges that use technologies such as RFID, Bluetooth low energy (BLE), MAC addresses from cell phones, NFC, etc. with captured video.

The process 30 executes on the one or more computers, and receives 32 sensory inputs from credentials or badges within a monitored premises. Sensors such as some of the nodes in FIG. 1 sense the presence of a credential tag typically carried by an individual passing through a range of the sensor. These sensors receive signals from badge/tag devices that incorporate circuitry operative using radio frequency identification (RFID), Bluetooth^{®} low energy peer to peer devices, MAC addresses from cell phones, and near field communication (NFC) devices operative using a set of standards for smartphones and similar devices to establish radio communication with each other by touching them together or bringing them into proximity, etc. that are dispersed throughout a monitored premises. The process 30 also receives 34 video information from cameras and other image capture devices that are disposed throughout the premises. The process 30 continually applies one or more algorithms to detect the presence of a possible non-credentialed individual to continually correlate the received sensory inputs from these credentials or badges, etc. with the received video.

The process 30 seeks to track individuals, especially individuals without valid credentials or badges for a particular monitored area. The process 30 applies one or more algorithms that detect the presence of a possible non-credentialed individual, tracks 38 at least the non-credentialed individual, alerts 40 authorities of the presence of a non-credentialed individual within the premises, and continually processing of inputs to isolate tracking of the non-credentialed individual to a particular, e.g., pinpoint location and alert 42 authorities to the isolated location of the non-credentialed individual.

During processing in FIG. 2, various ones of the nodes in FIG. 1 can be brought to bear on tracking of the non-credentialed individual or generally tracking of credentialed individuals. Thus, the servers of FIG. 1 or other systems can generate updates to functions (not shown) that are performed at the lower tiers, these nodes can receive 46 these new or updated functions and apply 48 the changed function to processing of FIG. 2.

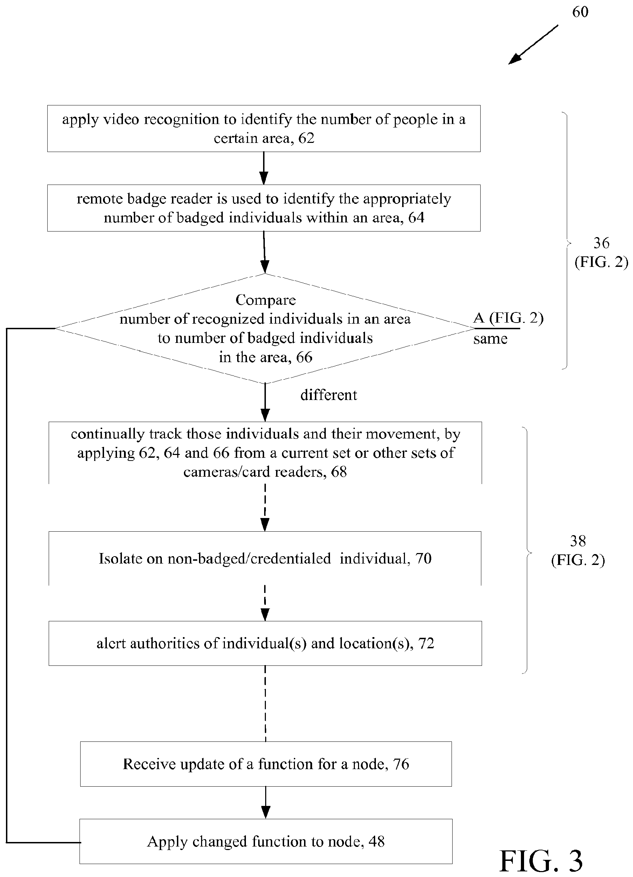

Referring now to FIG. 3 an example of algorithmic processing 60 for the tracking process 30 of FIG. 3, is shown. Cameras dispersed without the premises being monitored. Generally, the processing 36 of FIG. 2 is shown with details. One camera in an area captures video and applies 62 video recognition to frames of captured video. The video recognition is used to recognize features that correspond to, e.g., individuals appearing in the captured frames. The video recognition determines a number of people with the image, where the image is correlated to the area captured by the camera. At this junction the video recognition that is applied can be rather coarse to conserve processing or allow such processing to be performed of nodes within the lower tier, as it merely needs to find features that correspond to a number of individuals.

A remote badge reader that can be one of the nodes in the network 10 has a range that coincides, or overlaps or otherwise can be correlated to the area that was captured in the video frames. This remote badge reader and camera can be considered as a current set.

The computer(s) receives 64 badge/tag data from those individuals that are within the range of operation of the badge reader. The remote badge reader and/or computer(s) determines 66 the number of badged individuals that pass through the region within the range of operation of the remote badge reader. If the area captured by the remote badge reader coincides with the area captured by the camera, this data can be processed, otherwise they may be some correlation (not shown) need to correlate the area captured in the video frames with the area within the range of the card reader.

The computer compares the number of recognized individuals in frames to the number of received badges from the reader. If the process 60 determines that there is a mismatch between the number of individuals in the area and a number of badges (or credentials), the process 60 continues (generally, the processing 38 of FIG. 2) to track 68 all of those individuals and their movements throughout the premises by continually applying 62 video recognition to frames of captured video, receive 64 badge/tag and determining 66 the number of badged individuals that pass through the region within the range of badge readers with respect to the number of recognized individuals, using either the current set of cameras/readers or different sets of cameras/readers, as needed. At this juncture, the process could send requests for updated algorithms, as is discussed below for both the overall process (as in FIG. 2) or to update nodes for different node-level processing and sensing (FIG. 3).

The process 60 correlates the paths taken by different individuals with different readings of valid credentials or badges from the same or different sets of cameras/readers.

For example, at this junction more sophisticated recognition algorithms, e.g., facial recognition, etc. can be used. In addition, processing algorithms can be sent to other nodes in the network to train process on the tracked individuals where the nodes that are send these algorithms are selected based on an estimation or prediction of direction/paths of travel through the premises.

At some point as individuals come and go, a non-badged/credentialed individual can be isolated individually or to a small group, and then the process will focus tracking on that individual. At any point in processing where there is a discrepancy an alarm can be raised. As the process detects the presence of new individuals and detects the departure of previously tracked individuals from the group of individuals, the process is still continually tracking the one or more individuals without valid credentials. Newly added individuals can be recognized in the video captured, especially if more intensive algorithms are used, and departing individuals can be noted by a valid reading of their tags/credentials. If an individual departs without a valid tag read, when is should have been read, that person is most likely the non-credentialed individual.

As previously mentioned, using the network 10 of FIG. 1, it is possible during processing that various ones of the nodes in FIG. 1 are brought to bear on tracking of FIG. 2. Thus, the servers of FIG. 1 or other systems can generate updates to functions (not shown) that are performed at the lower tiers e.g., such nodes. These nodes at the lower tier receive 46 these new or updated functions and apply 48 the changed function to the processing performed by the nodes within processing of FIG. 2.

Examples of updated processing include sending more sophisticated recognition algorithms to video cameras or nodes that process the video information. Other examples are that certain ones of the nodes in FIG. 1 can be IP address reading sensors that are brought to bear on tracking of the non-credentialed individual or generally tracking of credentialed individuals.

Thus, the servers of FIG. 1 or other systems can generate updates to functions (not shown) that are performed at the lower tiers, these nodes can receive 46 these new or updated functions and apply 48 the changed function to processing of FIG. 2. As previously tracked individuals the depart, card readers can determine/sense credentials and the process can determine if they were validly credentialed and if so, terminate tracking on those departed individuals. On the other hand as new tracked individuals join, card readers can determine/sense credentials and the process can determine if they are validly credentialed and if so, terminate tracking an those new individuals. At some point the non-credentialed individual can be isolated to one or a few individuals, and his/their location(s) identified. The process produces and sends a message that notifies authorities to physically intervene with the message including available location information.

The nodes may be implemented using any appropriate type of computing device, such as a mainframe work station, a personal computer, a server, a portable computing device, or any other type of intelligent device capable of executing instructions, connecting to a network, and forwarding data packets through the network. The nodes can execute any appropriate computer programs to generate, receive, and transmit data packets for use on the network.

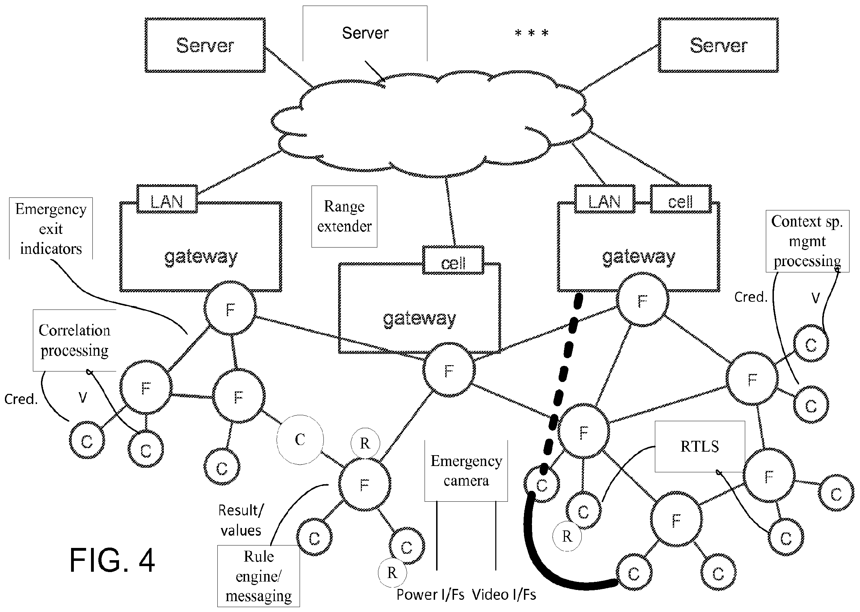

FIG. 4 shows an example of a security system having features of the WSN described with respect to FIGS. 1 to 3 and having the various functionalities described herein. As shown in FIG. 4, correlation processing receives inputs from certain constrained nodes (although these can also be fully functional nodes). These inputs may include credential information and video information, and the correlation processing may produce correlated results that are sent over the network. Context management processing receives inputs from certain constrained nodes (although these can also be fully functional nodes) e.g., credential information and video and grouping information, and performs context processing with results sent over the network. The network supports Operation of emergency exit indicators; emergency cameras as well as distributed rule processing and rule engine/messaging processing. Range extenders are used with e.g., gateways, and a real time location system receives inputs from various sensors (e.g., constrained type) as shown. Servers interface to the WSN via a cloud computing configuration and Parts of some networks can be run as subnets.

The sensors provide in addition to an indication that something is detected in an area within the range of the sensors, detailed additional information that can be used to evaluate what that indication may be without the intrusion detection panel being required to perform extensive analysis of inputs to the particular sensor.

For example, a motion detector could be configured to analyze the heat signature of a warm body moving in a room to determine if the body is that of a human or a pet. Results of that analysis would be a message or data that conveys information about the body detected. Various sensors thus are used to sense sound, motion, vibration, pressure, heat, images, and so forth, in an appropriate combination to detect a true or verified alarm condition at the intrusion detection panel.

Recognition software can be used to discriminate between objects that are a human and objects that are an animal; further facial recognition software can be built into video cameras and used to verify that the perimeter intrusion was the result of a recognized, authorized individual. Such video cameras would comprise a processor and memory and the recognition software to process inputs (captured images) by the camera and produce the metadata to convey information regarding recognition or lack of recognition of an individual captured by the video camera. The processing could also alternatively or in addition include information regarding characteristic of the individual in the area captured/monitored by the video camera. Thus, depending on the circumstances, the information would be either metadata received from enhanced motion detectors and video cameras that performed enhanced analysis on inputs to the sensor that gives characteristics of the perimeter intrusion or a metadata resulting from very complex processing that seeks to establish recognition of the object.

Sensor devices can integrate multiple sensors to generate more complex outputs so that the intrusion detection panel can utilize its processing capabilities to execute algorithms that analyze the environment by building virtual images or signatures of the environment to make an intelligent decision about the validity of a breach.

Memory stores program instructions and data used by the processor of the intrusion detection panel. The memory may be a suitable combination of random access memory and read-only memory, and may host suitable program instructions (e.g. firmware or operating software), and configuration and operating data and may be organized as a file system or otherwise. The stored program instruction may include one or more authentication processes for authenticating one or more users. The program instructions stored in the memory of the panel may further store software components allowing network communications and establishment of connections to the data network. The software components may, for example, include an internet protocol (IP) stack, as well as driver components for the various interfaces, including the interfaces and the keypad. Other software components suitable for establishing a connection and communicating across network will be apparent to those of ordinary skill.

Program instructions stored in the memory, along with configuration data may control overall operation of the panel.

The monitoring server includes one or more processing devices (e.g., microprocessors), a network interface and a memory (all not illustrated). The monitoring server may physically take the form of a rack mounted card and may be in communication with one or more Operator terminals (not shown). An example monitoring server is a SURGARD^{™} SG-System III Virtual, or similar system.

The processor of each monitoring server acts as a controller for each monitoring server, and is in communication with, and controls overall operation, of each server. The processor may include, or be in communication with, the memory that stores processor executable instructions controlling the overall operation of the monitoring server. Suitable software enable each monitoring server to receive alarms and cause appropriate actions to occur. Software may include a suitable Internet protocol (IP) stack and applications/clients.

Each monitoring server of the central monitoring station may be associated with an IP address and port(s) by which it communicates with the control panels and/or the user devices to handle alarm events, etc. The monitoring server address may be static, and thus always identify a particular one of monitoring server to the intrusion detection panels. Alternatively, dynamic addresses could be used, and associated with static domain names, resolved through a domain name service.

The network interface card interfaces with the network to receive incoming signals, and may for example take the form of an Ethernet network interface card (NIC). The servers may be computers, thin-clients, or the like, to which received data representative of an alarm event is passed for handling by human operators. The monitoring station may further include, or have access to, a subscriber database that includes a database under control of a database engine. The database may contain entries corresponding to the various subscriber devices/processes to panels like the panel that are serviced by the monitoring station.

All or part of the processes described herein and their various modifications (hereinafter referred to as "the processes") can be implemented, at least in part, via a computer program product, i.e., a computer program tangibly embodied in one or more tangible, physical hardware storage devices that are computer and/or machine-readable storage devices for execution by, or to control the Operation of, data processing apparatus, e.g., a programmable processor, a computer, or multiple computers. A computer program can be written in any form of programming language, including compiled or interpreted languages, and it can be deployed in any form, including as a stand-alone program or as a module, component, subroutine, or other unit suitable for use in a computing environment. A computer program can be deployed to be executed on one computer or on multiple computers at one site or distributed across multiple sites and interconnected by a network.

Actions associated with implementing the processes can be performed by one or more programmable processors executing one or more computer programs to perform the functions of the calibration process. All or part of the processes can be implemented as, special purpose logic circuitry, e.g., an FPGA (field programmable gate array) and/or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only storage area or a random access storage area or both. Elements of a computer (including a server) include one or more processors for executing instructions and one or more storage area devices for storing instructions and data. Generally, a computer will also include, or be operatively coupled to receive data from, or transfer data to, or both, one or more machine-readable storage media, such as mass storage devices for storing data, e.g., magnetic, magneto-optical disks, or optical disks.

Tangible, physical hardware storage devices that are suitable for embodying computer program instructions and data include all forms of non-volatile storage, including by way of example, semiconductor storage area devices, e.g., EPROM, EEPROM, and flash storage area devices; magnetic disks, e.g., internal hard disks or removable disks; magneto-optical disks; and CD-ROM and DVD-ROM disks and volatile computer memory, e.g., RAM such as static and dynamic RAM, as well as erasable memory, e.g., flash memory.

In addition, the logic flows depicted in the figures do not require the particular order shown, or sequential order, to achieve desirable results. In addition, other actions may be provided, or actions may be eliminated, from the described flows, and other components may be added to, or removed from, the described systems. Likewise, actions depicted in the figures may be performed by different entities or consolidated.

Elements of different embodiments described herein may be combined to form other embodiments not specifically set forth above. Elements may be left out of the processes, computer programs, Web pages, etc. described herein without adversely affecting their operation. Furthermore, various separate elements may be combined into one or more individual elements to perform the functions described herein.

## Claims

1. A security system of a building, the system comprising:
- one or more computing devices, the one or more computing devices comprising one or more processor devices and one or more memory devices in communication with the one or more processor devices, wherein the one or more processor devices are configured to:
- receive, from reader devices, sensory inputs associated with credentials or badges, wherein the reader devices are associated with one or more first areas of the building;
- determine the number of badged or credentialed individuals that pass through the first area;
- receive video data from camera devices, wherein the video data comprises a video feed of one or more second areas of the building, wherein the second areas coincide, or overlap or otherwise can be correlated to the first areas;
- continually correlate sensory inputs of the reader devices with the video data of the camera devices;
- determine, from the video data, a number of individuals within the one or more first areas of the building by applying video recognition to the video data;
- determine a mismatch between the number of individuals and the number of read credentials or badges;
- track a group of individuals while moving through the building based on the video data and the sensory inputs in response to the determination of the mismatch between the number of individuals and the number of credentials or badges;
- determine that one or more individuals of the group of individuals is a validly credentialed individual by determining, based on the video data and the sensory inputs of the reader devices, that one or more individuals of the group of individuals has departed the building with a valid credential or a valid badge; and
- determine that one or more individuals of the group of individuals is not a validly credentialed individual by determining, based on the video data and the sensory inputs of the reader devices, that one or more individuals of the group of individuals has departed the building without a valid credential or a valid badge.

2. The system of claim 1,
wherein the one or more computing devices are configured to dynamically change first algorithms configured to detect presence of a particular number of the one or more individuals to second algorithms configured to apply video recognition to recognize the one or more individuals.

3. The system of claim 1 or 2,
wherein the one or more computing devices are configured to dynamically update a video recognition algorithm to find features to a particular number of the one or more individuals.

4. The system of one of claims 1 to 3,
wherein the one or more computing devices are configured to:
- dynamically change recognition algorithms; and
- correlate paths taken by different individuals with different readings of valid credentials or valid badges from a same or different set of the camera devices and the reader devices.

5. The system of one of claims 1 to 4,
wherein the one or more computing devices are configured to dynamically change one or more algorithms of the one or more computing devices while tracking the group of the individuals by the one or more computing devices by executing an operating system allowing loading and execution of individual algorithms after an initial booting stage without requiring a rebooting of the one or more computing devices to dynamically change the one or more algorithms.

6. The system of one of claims 1 to 5,
wherein the one or more processor devices are configured to:
- continually track the group of the one or more individuals and movements of the group of the one or more individuals throughout the monitored premises; and
- correlate the movements of the group of the one or more individuals with different readings of valid credentials or valid badges to isolate one or more unauthorized individuals.

7. The system of one of claims 1 to 6,
wherein the one or more processor devices are configured to:
- apply one or more recognition algorithms to the video data to coarsely recognize features identifying the number of the one or more individuals; and
- produce an alert to send a control center to alert authorities to a particular location of a non-credentialed individual, the particular location being based on device locations of the camera devices and the reader devices.

8. The system of one of claims 1 to 7,
wherein the one or more processor devices are configured to apply video recognition to identify the number of the one or more individuals.

9. The system of one of claims 1 to 8,
wherein the system receives updated routines from server systems, the updated routines being updates for recognition algorithms.

10. The system of one of claims 1 to 9, configured to correlate paths taken by different individuals with different readings of valid credentials or valid badges from same or different sets of the reader devices and the camera devices.

## Patentansprüche

1. Sicherheitssystem eines Gebäudes, wobei das System umfasst:
- eine oder mehrere Rechenvorrichtungen, wobei die eine oder mehreren Rechenvorrichtungen eine oder mehrere Prozessorvorrichtungen und eine oder mehrere Speichervorrichtungen in Kommunikation mit der einen oder den mehreren Prozessorvorrichtungen umfassen, wobei die eine oder mehreren Prozessorvorrichtungen dazu konfiguriert sind:
- von Lesevorrichtungen sensorische Reize zu empfangen, welche mit Berechtigungsnachweisen oder Ausweisen verknüpft sind, wobei die Lesevorrichtungen mit einem oder mehreren ersten Bereichen des Gebäudes verknüpft sind;
- die Zahl der ausgewiesenen oder berechtigten Personen zu bestimmen, welche durch den ersten Bereich gelangen;
- Videodaten aus Kameravorrichtungen zu empfangen, wobei die Videodaten einen Videofeed eines oder mehrerer zweiter Bereiche des Gebäudes umfassen, wobei die zweiten Bereiche mit den ersten Bereichen zusammenfallen, überlappen oder auf andere Weise damit korreliert werden können;
- sensorische Reize der Lesevorrichtungen kontinuierlich mit den Videodaten der Kameravorrichtungen zu korrelieren;
- aus den Videodaten durch Anwenden von Videoerkennung auf die Videodaten eine Zahl von Personen innerhalb des einen oder der mehreren ersten Bereich des Gebäudes zu bestimmen;
- eine Diskrepanz zwischen der Zahl von Personen und der Zahl von gelesenen Berechtigungsnachweisen oder Ausweisen zu bestimmen;
- als Reaktion auf die Bestimmung der Diskrepanz zwischen der Zahl von Personen und der Zahl von Berechtigungsnachweisen oder Ausweisen eine Gruppe von Personen auf Basis der Videodaten und der sensorischen Reize während der Bewegung durch das Gebäude zu verfolgen;
- durch Bestimmen auf Basis der Videodaten und der sensorischen Reize der Lesevorrichtungen, dass eine oder mehrere Personen der Gruppe von Personen das Gebäude mit einem gültigen Berechtigungsnachweise oder einem gültigen Ausweises verlassen hat, zu bestimmen, dass eine oder mehrere Personen der Gruppe von Personen eine gültig berechtigte Person ist; und
- durch Bestimmen auf Basis der Videodaten und der sensorischen Reize der Lesevorrichtungen, dass eine oder mehrere Personen der Gruppe von Personen das Gebäude ohne einem gültigen Berechtigungsnachweise oder einem gültigen Ausweises verlassen hat, zu bestimmen, dass eine oder mehrere Personen der Gruppe von Personen keine gültig berechtigte Person ist.

2. System nach Anspruch 1,
wobei die eine oder mehreren Rechenvorrichtungen dazu konfiguriert sind, erste Algorithmen, welche dazu konfiguriert sind, um Anwesenheit einer bestimmten Zahl der einen oder mehreren Personen zu erfassen, dynamisch in zweite Algorithmen zu ändern, welche dazu konfiguriert sind, Videoerkennung anzuwenden, um die eine oder mehreren Personen zu erkennen.

3. System nach Anspruch 1 oder 2,
wobei die eine oder mehreren Rechenvorrichtungen dazu konfiguriert sind, einen Videoerkennungsalgorithmus dynamisch zu aktualisieren, um Merkmale für eine bestimmte Zahl der einen oder mehreren Personen zu finden.

4. System nach einem der Ansprüche 1 bis 3,
wobei die eine oder mehreren Rechenvorrichtungen dazu konfiguriert sind:
- Erkennungsalgorithmen dynamisch zu ändern; und
- Wege, welche von unterschiedlichen Personen genommen wurden, mit unterschiedlichen Ablesungen von gültigen Berechtigungsnachweisen oder gültigen Ausweisen aus einem selben oder unterschiedlichen Satz der Kameravorrichtungen und der Lesevorrichtungen zu korrelieren.

5. System nach einem der Ansprüche 1 bis 4,
wobei die eine oder mehreren Rechenvorrichtungen dazu konfiguriert sind, einen oder mehrere Algorithmen der einen oder mehreren Rechenvorrichtungen während dem Verfolgen der Gruppe der Person durch die eine oder mehreren Rechenvorrichtungen durch Ausführen eines Betriebssystems, welches Laden und Ausführen von einzelnen Algorithmen nach einer anfänglichen Bootphase erlaubt, ohne ein Reboot der einen oder mehreren Rechenvorrichtungen zu erfordern, dynamisch zu ändern, um den einen oder die mehreren Algorithmen dynamisch zu ändern.

6. System nach einem der Ansprüche 1 bis 5,
wobei die eine oder mehreren Prozessorvorrichtungen dazu konfiguriert sind:
- die Gruppe der einen oder mehreren Personen und Bewegungen der Gruppe der einen oder mehreren Personen durch die überwachten Räumlichkeiten kontinuierlich zu verfolgen; und
- die Bewegungen der Gruppe der einen oder mehreren Personen mit unterschiedlichen Ablesungen von gültigen Berechtigungsnachweisen oder gültigen Ausweisen zu korrelieren, um eine oder mehrere unbefugte Personen zu isolieren.

7. System nach einem der Ansprüche 1 bis 6,
wobei die eine oder mehreren Prozessorvorrichtungen dazu konfiguriert sind:
- einen oder mehrere Erkennungsalgorithmen auf die Videodaten anzuwenden, um grob Merkmale zu erkennen, welche die Zahl der einen oder mehreren Personen identifizieren; und
- eine Warnung zu erzeugen, welche an eine Leitstelle gesendet werden soll, um Zuständige auf einen bestimmten Standort einer nicht berechtigten Person aufmerksam zu machen, wobei der bestimmte Standort auf Standorten von Vorrichtungen der Kameravorrichtungen und der Lesevorrichtungen basiert.

8. System nach einem der Ansprüche 1 bis 7,
wobei die eine oder mehreren Prozessorvorrichtungen dazu konfiguriert sind, Videoerkennung anzuwenden, um die Zahl der einen oder mehreren Personen zu identifizieren.

9. System nach einem der Ansprüche 1 bis 8,
wobei das System aktualisierte Abläufe von Serversystemen empfängt, wobei die aktualisierten Abläufe Aktualisierungen für Erkennungsalgorithmen sind.

10. System nach einem der Ansprüche 1 bis 9, welches dazu konfiguriert ist, Wege, welche von unterschiedlichen Personen genommen wurden, mit unterschiedlichen Ablesungen von gültigen Berechtigungsnachweisen oder gültigen Ausweisen aus einem selben oder unterschiedlichen Sätzen der Lesevorrichtungen und der Kameravorrichtungen zu korrelieren.

## Revendications

1. Système de sécurité d'un bâtiment, le système comprenant :
- un ou plusieurs dispositifs informatiques, les un ou plusieurs dispositifs informatiques comprenant un ou plusieurs dispositifs processeurs et un ou plusieurs dispositifs mémoire en communication avec les un ou plusieurs dispositifs processeurs, dans lequel les un ou plusieurs dispositifs processeurs sont configurés pour :
- recevoir, à partir de dispositifs de lecture, des entrées sensorielles associées à des identifiants ou des badges, dans lequel les dispositifs de lecture sont associés à une ou plusieurs premières zones du bâtiment ;
- déterminer le nombre de personnes badgées ou identifiées qui transitent par la première zone ;
- recevoir des données vidéo provenant de dispositifs de caméra, dans lequel les données vidéo comprennent un flux vidéo d'une ou de plusieurs secondes zones du bâtiment, dans lequel les secondes zones coïncident, se chevauchent ou peuvent être corrélées aux premières zones ;
- corréler continuellement les entrées sensorielles des dispositifs de lecture avec les données vidéo des dispositifs de caméra ;
- déterminer, à partir des données vidéo, un nombre d'individus dans les une ou plusieurs premières zones du bâtiment en appliquant une reconnaissance vidéo aux données vidéo ;
- déterminer une inadéquation entre le nombre d'individus et le nombre d'identifiants ou de badges lus ;
- suivre un groupe d'individus tout en se déplaçant dans le bâtiment sur la base des données vidéo et des entrées sensorielles en réponse à la détermination de l'inadéquation entre le nombre d'individus et le nombre d'identifiants ou de badges ;
- déterminer qu'un ou plusieurs individus du groupe d'individus sont des individus valablement identifiés en déterminant, sur la base des données vidéo et des entrées sensorielles des dispositifs de lecture, qu'un ou plusieurs individus du groupe d'individus ont quitté le bâtiment avec un identifiant ou un badge valide ; et
- déterminer qu'un ou plusieurs individus du groupe d'individus ne sont pas des individus valablement identifiés en déterminant, sur la base des données vidéo et des entrées sensorielles des dispositifs de lecture, qu'un ou plusieurs individus du groupe d'individus ont quitté le bâtiment sans un identifiant ou un badge valide.

2. Système selon la revendication 1,
dans lequel les un ou plusieurs dispositifs informatiques sont configurés pour changer dynamiquement des premiers algorithmes configurés pour détecter la présence d'un nombre particulier des un ou plusieurs individus en seconds algorithmes configurés pour appliquer une reconnaissance vidéo pour reconnaître les un ou plusieurs individus.

3. Système selon la revendication 1 ou 2,
dans lequel les un ou plusieurs dispositifs informatiques sont configurés pour mettre à jour dynamiquement un algorithme de reconnaissance vidéo afin de trouver des caractéristiques pour un nombre particulier des un ou plusieurs individus.

4. Procédé selon l'une des revendications 1 à 3,
dans lequel les un ou plusieurs dispositifs informatiques sont configurés pour :
- modifier dynamiquement les algorithmes de reconnaissance ; et
- corréler les chemins empruntés par différentes personnes avec différentes lectures d'identifiants valides ou de badges valides provenant d'un ensemble identique ou différent de dispositifs de caméra et de dispositifs de lecture.

5. Procédé selon l'une des revendications 1 à 4,
dans lequel les un ou plusieurs dispositifs informatiques sont configurés pour modifier dynamiquement un ou plusieurs algorithmes des un ou plusieurs dispositifs informatiques tout en suivant le groupe d'individus par les un ou plusieurs dispositifs informatiques en exécutant un système d'exploitation permettant le chargement et l'exécution d'algorithmes individuels après une étape de démarrage initiale sans nécessiter un redémarrage des un ou plusieurs dispositifs informatiques pour modifier dynamiquement les un ou plusieurs algorithmes.

6. Procédé selon l'une des revendications 1 à 5,
dans lequel les un ou plusieurs dispositifs processeurs sont configurés pour:
- suivre en permanence le groupe des un ou plusieurs individus et les mouvements du groupe des un ou plusieurs individus dans l'ensemble des locaux surveillés ; et
- corréler les mouvements du groupe des un ou plusieurs individus avec différentes lectures d'identifiants valides ou de badges valides pour isoler un ou plusieurs individus non autorisés.

7. Procédé selon l'une des revendications 1 à 6,
dans lequel les un ou plusieurs dispositifs processeurs sont configurés pour:
- appliquer un ou plusieurs algorithmes de reconnaissance aux données vidéo pour reconnaître grossièrement des caractéristiques identifiant le nombre des un ou plusieurs individus ; et
- produire une alerte pour envoyer un centre de contrôle pour alerter les autorités d'un emplacement particulier d'un individu non identifié, l'emplacement particulier étant basé sur des emplacements de dispositif des dispositifs de caméra et des dispositifs de lecture.

8. Procédé selon l'une des revendications 1 à 7,
dans lequel les un ou plusieurs dispositifs processeurs sont configurés pour appliquer une reconnaissance vidéo afin d'identifier le nombre des un ou plusieurs individus.

9. Procédé selon l'une des revendications 1 à 8,
dans lequel le système reçoit des routines mises à jour en provenance de systèmes serveurs, les routines mises à jour étant des mises à jour pour des algorithmes de reconnaissance.

10. Système selon l'une des revendications 1 à 9, configuré pour corréler des chemins empruntés par différents individus avec différentes lectures d'identifiants valides ou de badges valides provenant d'ensembles identiques ou différents de dispositifs de lecture et de dispositifs de caméra.
